# EUROPEAN PATENT APPLICATION

(11) **EP 2 526 901 A1**
(43) Date of publication of application: **28.11.2012**
(21) Application number: 12165655.7
(22) Date of filing: 26.04.2012
(51) Int. Cl.: A61F 2/30

(54) **Method for manufacturing a medical implant with a radiopaque marker**

(30) Priority: 17.05.2011 GB 201108185
(71) Applicant: DePuy Spine Sàrl, 2400 Le Locle (CH)
(72) Inventor: Spratt, Frank, 2400 Le Locle (CH); Betts, Duncan, 2400 Le Locle (CH); Magliano, Giancarlo, 2400 Le Locle (CH)
(74) Representative: Brown, George Laurence

(57) **Abstract**

A method of manufacturing a medical implant comprising a radiopaque marker is described. The method comprises manufacturing a medical implant using stereolithography, wherein the medical implant has an external surface that delimits an opening to a channel. A curable mixture of a biocompatible polymer and a radiopaque material is then inserted into the channel while in a liquid state and cured in the channel so that it solidifies in the channel.

The combination of an implant manufactured by stereolithography and a radiopaque marker which is inserted into the channel in a liquid state enables complex internal structures to be formed in the implant for the radiopaque marker and for the marker to take on those complex forms more easily. This allows a better visualisation of the marker under X-Ray or other medical imaging technique.

## Description

The present application relates to a medical implant comprising at least one radiopaque marker, a method for manufacturing a medical implant including at least one radiopaque marker and a curable mixture for forming a radiopaque marker in a medical implant.

Medical implants are used in many surgical procedures. Examples of such procedures include orthopaedic surgery, such as spinal surgery.

Recently, the use of polyetheretherketone (PEEK) has been proposed for use in medical implants. PEEK and PEEK composites are biocompatible and have good mechanical properties. However, PEEK has a disadvantage because it is radio translucent, so that PEEK and PEEK composite based products are not easily visible on X-rays and other imaging techniques.

To improve visibility of an implant under X-ray, it is known to provide a medical implant with radiopaque markers. The radiopaque markers increase visibility under X-ray and other imaging techniques. Typically, these radiopaque markers are formed from beads and wires of a radiopaque material. The beads and wires are press-fitted into openings formed in the product. The use of radiopaque beads and wires in this way has several disadvantages. Firstly, the tolerance of the opening for the marker and the size of the marker itself must be very tight to ensure that a suitably strong interference fit can be achieved. Difficulties in achieving these tolerances mean that sometimes 5% or more of the medical implants manufactured have to be rejected due to difficulties with the press-fit connection of the radiopaque marker.

US-A-2001/0027343 (Keller) discusses a plastic implant which is provided with a circumferential channel for receiving a radiographic contrast wire. The channel is at least partially closed to retain the wire in place. The wire forms an open loop within the channel.

The use of wires and beads to form the radiopaque marker also has a disadvantage that when viewed on an X-ray or other imaging technique, only the markers are easily visible. The beads appear as points and the wires must follow straight lines or have constant radius of curvature as discussed in Keller. This results in a relatively poor representation of the product when imaged.

US-A-2008/0234532 (De Langen et al) discusses a radiopaque fiducial marker. This is a standalone fiducial marker which is used to indicate reference points during surgery. The fiducial marker can comprise a radiopaque material encapsulated in a biocompatible polymeric material. The fiducial marker of De Langen et al is not incorporated into a medical implant. In one example discussed in De Langen et al, the fiducial marker is produced by compounding PEEK polymer and barium sulphate powder in a twin screw melt extrusion compounder. This technique is not appropriate for manufacture of medical implants.

It would be desirable to provide a medical implant with radiopaque markers which can better indicate the shape and form of the implant under X-ray or other imaging technique. It would also be desirable to improve the ease with which a medical implant comprising a radiopaque marker can be manufactured.

Accordingly, one aspect of the present invention provides a method for manufacturing a medical implant comprising a radiopaque marker, in which the radiopaque marker is formed from a curable mixture of a biocompatible polymer and a radiopaque material. This allows the radiopaque mixture to be inserted into the medical implant in liquid form, removing the difficulties with press-fitting in production and allowing more complex shapes of radiopaque marker to be formed.

According to another aspect of the invention, a medical implant comprising a radiopaque marker which includes at least one turn is provided. The radiopaque marker incorporates a turn, and so can indicate the shape of the implant under X-ray or other visualisation technique more accurately than prior art methods using straight wire or beads.

According to an aspect of the present invention, there is provided a method of manufacturing a medical implant comprising a radiopaque marker, the method comprising:
providing a medical implant using stereolithography, wherein the medical implant has an external surface that delimits an opening to a channel;
inserting a curable mixture of a biocompatible polymer and a radiopaque material into the channel while in a liquid state; and
curing the curable mixture in the channel so that it solidifies in the channel.

The curable mixture is inserted into the channel in a liquid state. A liquid state is a condition in which the mixture can flow, i.e. the mixture is not a solid. This overcomes problems with tight tolerances required for press-fitting in the prior art. As a further advantage, it allows the channel to take on more complex shapes than the prior art, for example using curved channels and/or channels which include at least one turn. The liquid nature of the curable mixture when it is inserted means that it conforms to the shape of the channel.

The medical implant is manufactured by stereo lithography. Stereo lithography is an additive manufacturing technology whereby multiple layers of material are formed successively to create a three dimensional structure. Manufacturing the implant by stereo lithography enables complex internal structures to be formed, such as curved channels and channels including turns. The combination of these complex internal structures with the radiopaque marker created in this invention enables improved visualisation of the form of the medical implant under X-ray or other medical imaging technique.

The medical implant can be any implant for use in the human or animal body. Preferably, it is a permanent implant, for example an orthopaedic implant. The medical implant may be for implantation at the interface of bone surfaces, for example between vertebra or any other articulating bone joint, such as a knee.

Preferably the channel follows a path comprising at least one turn. A "turn" is any change of direction of the channel, including curved portions and instantaneous turns, in other words the channel does not have constant radius of curvature along its length and follows a path which is not a straight line. If the channel follows a path comprising at least one turn it can indicate the shape of the implant more accurately under X-ray or other imaging technique.

The channel may be a groove formed in the surface of the implant. More preferably, the channel may extend into the medical implant, effectively defining a passageway or other opening within the implant. By providing the channel within the implant, any subsequent surface processing of the radiopaque marker on the surface of the implant is minimised. In that case, the channel preferably terminates within the medical implant, i.e. it has only one opening at the surface of the medical implant. Such a channel can easily be filled with a radiopaque marker in liquid form.

The channel preferably has a transverse dimension of at least about 1mm. For example, if the channel has a generally circular cross-section, the diameter of the cross-section may be about 1 mm or greater. The channel may have any suitable length depending on the requirements for the implant, but is preferably between about 5mm and about 30mm.

Preferably, the channel follows a path comprising a first turn and a second turn. This allows the channel to follow a more complex path and indicate the form of the implant more accurately.

In embodiments of the invention, the first turn may change the heading of the channel by a different amount than the second turn. The first turn may have a first radius of curvature and the second turn may have a second radius of curvature which is not equal to the first radius of curvature. The first turn may have a first arc length and the second turn may have a second arc length which is not equal to the first arc length. These embodiments allow the channel to follow more complex paths and more closely indicate the shape of the medical implant.

In other embodiments the channel may follow a path which is at least partially curved. In the prior art, curved paths cannot be achieved because of using a press-fit technique requires straight lines.

The more complex forms of the channel of the present invention allow the channel to take a variety of shapes and paths. For example, in some embodiments the channel may define a unique identifier for the implant. This could be at the level of an individual product number, enabling the model of implant to be identified without reference to patient records. Alternatively or in addition it may include further additional information such as batch numbers and/or a unique serial number if required.

The curable mixture may be inserted by pouring it into the channel. Alternatively, depending on the viscosity of the curable mixture, it may be injected into the channel. The channel may be partially or completely filled by the curable mixture.

The curable mixture may be created by mixing a biocompatible polymer in liquid form with a radiopaque powder. This disperses the radiopaque powder throughout the biocompatible polymer so that when the curable mixture solidifies, a radiopaque marker is formed along the length of the channel.

The biocompatible polymer in liquid form may comprise any suitable biocompatible polymer. In some embodiments, it preferably comprises silicone or epoxy. In preferable embodiments, the biocompatible polymer may have adhesive properties, enhancing its adhesion into the channel when it solidifies. For example, in one embodiment, the biocompatible polymer in liquid form may comprise silicone adhesive and xylene.

The radiopaque powder may comprise tantalum or barium. Other radiopaque materials may also be used.

Preferably, the radiopaque material comprises between 25 and 40 weight % of the mixture. More preferably, the radiopaque material comprises between 30 and 35 weight % of the mixture.

In one embodiment, the biocompatible polymer in liquid form and the radiopaque powder are mixed for two minutes or less. Increased mixing time may cause the mixture to begin curing and become more viscous and/or develop an undesirable "skin" before it is inserted in the channel. Therefore, it is preferable to reduce the mixing time as much as possible, providing that an even mixture is still obtained.

To further reduce the effect of curing before the mixture is inserted into the medical implant, it is preferable that the mixing takes place in a sealed container to avoid the effects of the atmosphere. In other embodiments, the mixing may take place in a controlled atmosphere to limit any curing reaction which may take place during mixing.

According to a further aspect of the present invention, there is provided a medical implant comprising at least one radiopaque marker, wherein an external surface of the implant delimits an opening to a channel; the channel contains a quantity of a mixture comprising a biocompatible polymer and a radiopaque material. In some embodiments the channel may be completely filled with the mixture. Such an implant can be manufactured more easily than prior art implants relying on a press-fitted radiopaque marker.

The radiopaque material is preferably a radiopaque powder dispersed within the biocompatible polymer. More preferably, the radiopaque powder is evenly distributed throughout the biocompatible polymer. This embodiment provides a radiopaque marker which has substantially the same appearance under X-ray or imaging along the length of the channel. A further advantage is that a radiopaque marker which comprises a biocompatible polymer in addition to the radiopaque material, has a reduced cost compared with producing the radiopaque marker solely from radiopaque material.

The radiopaque material preferably comprises tantalum or barium. However, any suitable radiopaque material, which is radiopaque when illuminated under X-ray or other medical imaging technique, can be used. Examples include Iodine, barium salts, such as barium sulphate, amongst others.

Preferably, the biocompatible polymer comprises silicone or epoxy. Both these polymers can be provided in a curable liquid form, enabling the mixture of biocompatible polymer and radiopaque material to be applied to the implant as a liquid and then solidify when in place. This improves the ease of manufacture to insert the radiopaque marker into the channel in an implant.

According to another aspect of the present invention, there is provided a curable mixture for forming a radiopaque marker in a medical implant, the curable mixture comprising a biocompatible polymer in liquid form and a radiopaque material.

Preferably the radiopaque material is a powder.

Preferably the biocompatible polymer comprises silicone or epoxy. Preferably the radiopaque material comprises tantalum or barium.

The features of the above described aspects may be combined.

Embodiments of the invention will now be described by way of example and not limitation with reference to the accompanying drawings, in which:
Figure 1 depicts a perspective view of a spinal implant which may incorporate radiopaque markers according to the present invention;
Figure 2 is a diagrammatic representation of a simulated X-ray of the medical implant of

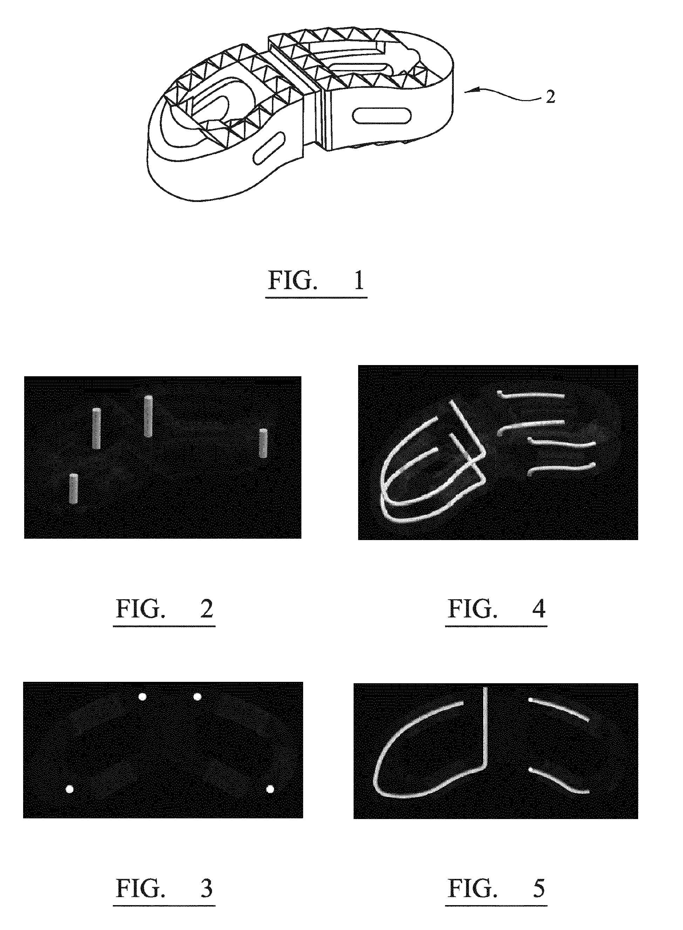

Figure 1 with prior art wire-based radiopaque markers;
Figure 3 depicts a diagrammatic representation of a simulated X-ray of the spinal implant of Figure 1 using radiopaque markers according to the present invention;
Figure 4 is a diagrammatic representation of a simulated X-ray of the medical implant of
Figure 1 with prior art wire-based radiopaque markers, viewed from above;
Figure 5 Figure 3 depicts a diagrammatic representation of a simulated X-ray of the spinal implant of Figure 1 using radiopaque markers according to the present invention, viewed from above;

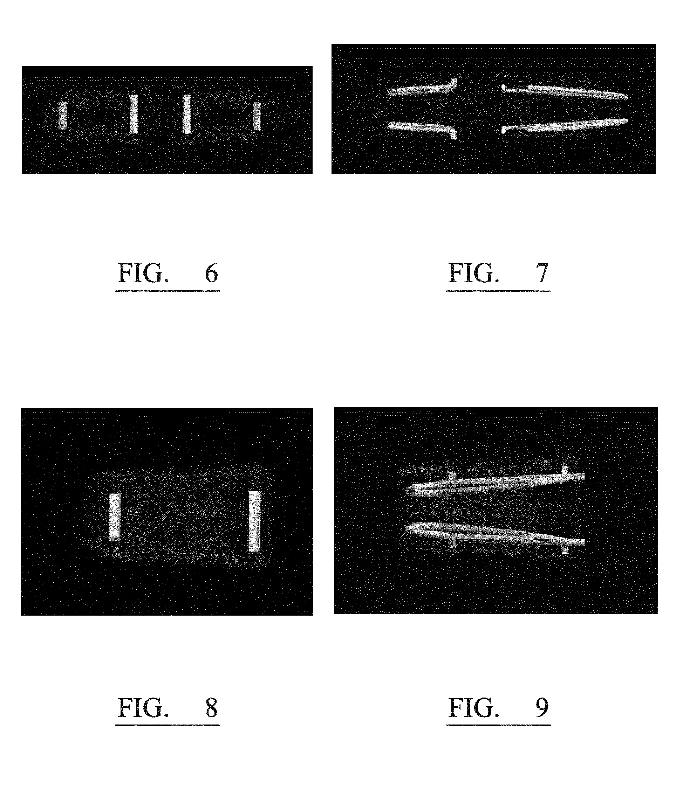

Figure 6 is a diagrammatic representation of a simulated X-ray of the medical implant of Figure 1 with prior art wire-based radiopaque markers, viewed from the front;
Figure 7 depicts a diagrammatic representation of a simulated X-ray of the spinal implant of Figure 1 using radiopaque markers according to the present invention, viewed from the front;
Figure 8 is a diagrammatic representation of a simulated X-ray of the medical implant of Figure 1 with prior art wire-based radiopaque markers, viewed from the side;
Figure 9 depicts a diagrammatic representation of a simulated X-ray of the spinal implant of Figure 1 using radiopaque markers according to the present invention, viewed from the side;

Figure 1 depicts an example of a spinal implant 2, which can be provided with radiopaque markers according to the present invention.

Figures 2, 4, 6 and 8 depict simulated X-rays of the spinal implant 2 using prior art radiopaque wire based markers which are press-fit into openings in the implant. As can be seen, these markers are limited to straight lines, resulting in a poor representation of the medical implant 2 under X-ray.

Figures 3, 5, 7 and 9 depict simulated X-rays of the medical implant 2 incorporating radiopaque markers according to the present invention. As can be seen, these markers include curved portions and/or turns defining complex paths that enable the shape of the implant to be determined more readily under X-ray.

In order to manufacture the complex channels within the medical implant 2, the medical implant 2 is preferably manufactured by an additive manufacturing technique, for example, stereo lithography. In stereo lithography, the shape of the implant is built up in layers according to conventional techniques known in the art. Because the structure is built up in layers, complex internal structures, for example as depicted for the radiopaque marker in Figures 3, 5, 7 and 9, can be manufactured.

In an example method of manufacturing the medical implant with radiopaque markers as depicted in Figures 3, 5, 7 and 9 the medical implant is first manufactured using known stereo lithography to include channels having turns and curves as discussed above.

Next, a curable liquid mixture of a biocompatible polymer and radiopaque material is prepared for application into the channels. In this example, the mixture may be based on implant grade dimethyl silicone dispersion in xylene or other products such as MED-1137 commercially available from Newsil. These products are biocompatible and suitable for use including long term human implantation. Radiopaque material is then added to the liquid before it begins curing.

In one embodiment, the mixture comprises 10±0.2g of silicone adhesive, 5±0.2g of xylene and 7±0.2g of tantalum powder.

The components are mixed by firstly measuring the required weight of silicone adhesive into a container, for example, using a balance. To this, the xylene is added into the container, for example weighed using a balance. Finally, the tantalum powder is added to the dispenser container, again weighed using a balance. The components are then mixed, preferably for a minimum of 30 seconds and a maximum of two minutes. This ensures that the components are well mixed and avoids problems due to overmixing. Overmixing may lead to air being trapped inside the mixture, and increases the possibility that the curing reaction may start, causing a skin to form on the mixture. Once mixed the curable liquid mixture is inserted into the channels by pouring or injecting, depending on its viscosity.

The insertion of the curable liquid mixture can be adapted to different forms of implants as required on a production line. This allows production machinery to be more versatile than existing press-fit machinery, which requires precise lengths of radiopaque marker wire and accurate alignment for the press-fit.

Thus, according to the invention, a medical implant can be formed which has improved visibility under X-ray or other medical imaging techniques. Furthermore, the medical implant can be manufactured more easily than prior art techniques, and can include more complex radiopaque marker shapes.

## Claims

1. A method of manufacturing a medical implant comprising a radiopaque marker, the method comprising:
manufacturing a medical implant using stereolithography, wherein the medical implant has an external surface that delimits an opening to a channel;
inserting a curable mixture of a biocompatible polymer and a radiopaque material into the channel while in a liquid state; and
curing the curable mixture in the channel so that it solidifies in the channel.

2. A method according to claim 1, wherein in the step of manufacturing the channel extends into the medical implant.

3. A method according to claim 2, wherein in the step of manufacturing the channel terminates within the medical implant.

4. A method according to any one of the preceding claims wherein the channel follows a path comprising at least a first turn and a second turn.

5. A method according to any one of claims 1 to 4, wherein the curable mixture is inserted by pouring it into the channel;

6. A method according to any one of claims 1 to 4, wherein the curable mixture is inserted by injecting it into the channel;

7. A method according to any one of the preceding claims, wherein the curable mixture is created by mixing a biocompatible polymer in liquid form with a radiopaque powder.

8. A method according to claim 7, wherein the biocompatible polymer in liquid form comprises silicone or epoxy.

9. A method according to claim 8, wherein the biocompatible polymer in liquid form comprises silicone adhesive and xylene.

10. A method according to claim 8 or 9, wherein the radiopaque powder comprises tantalum or barium.

11. A method according to any one of claims 7 to 10, wherein the biocompatible polymer in liquid form and the radiopaque powder are mixed for two minutes or less.

12. A method according to any one of claims 7 to 11, wherein the mixing takes place in a sealed container.

13. A method according to any one of the preceding claims, wherein the radiopaque material comprises between 25 and 40 weight % of the mixture.
